# EUROPÄISCHE PATENTSCHRIFT

(11) **EP 3 616 518 B1**
(45) Veröffentlichungstag und Bekanntmachung des Hinweises auf die Patenterteilung: **21.05.2025**
(21) Anmeldenummer: 19194118.6
(22) Anmeldetag: 28.08.2019
(51) Int. Cl.: A01N 47/46, A01N 25/08, A01P 1/00, A61K 31/26, A61P 31/04, A61P 31/12

(54) **ANTIMIKROBIELLES MITTEL ENTHALTEND ISOTHIOCYANATE**
ANTIMICROBIAL AGENT CONTAINING ISOTHIOCYANATES
AGENT ANTIMICROBIEN COMPRENANT DES ISOTHIOCYANATES

(30) Priorität: 30.08.2018 DE 102018121251
(43) Veröffentlichungstag der Anmeldung: 04.03.2020
(73) Patentinhaber: Shenstone Consultants Ltd., Shenstone Lichfield Staffordshire WS14 0ED (GB)
(72) Erfinder: Lee, Chris, Lichfield, Staffordshire WS14 0JG (GB)
(74) Vertreter: Schneiders & Behrendt Bochum

(56) Entgegenhaltungen:
- EP-A1- 1 961 418
- CN-A- 102 697 764
- .: "Fachinformation (Zusammenfassung der Merkmale des Arzneimittels) ANGOCIN Anti-Infekt N", 1 July 2015 (2015-07-01), Germany, pages 1 - 3, XP055640574, Retrieved from the Internet <URL:https://s3.eu-central-1.amazonaws.com/prod-cerebro-ifap/media_all/80997.pdf> [retrieved on 20191108]
- .: "OTC-Beratungskarte 76 Angocin Anti-Infekt N Filmtabletten", 6 September 2017 (2017-09-06), Germany, pages 1 - 2, XP055640568, Retrieved from the Internet <URL:https://www.deutschesapothekenportal.de/download/public/beratungskarten/dap_beratungskarte_angocin.pdf> [retrieved on 20191108]
- ANDREAS CONRAD ET AL: "In-vitro-Untersuchungen zur antibakteriellen Wirksamkeit einer Kombination aus Kapuzinerkressenkraut (Tropaeoli majoris herba) und Meerrettichwurzel (Armoraciae rusticanae radix)", ARZNEIM.-FORSCH./DRUG RES, 1 January 2006 (2006-01-01), Germany, pages 842 - 849, XP055640615, Retrieved from the Internet <URL:https://www.thieme-connect.com/products/ejournals/pdf/10.1055/s-0031-1296796.pdf> [retrieved on 20191108]
- KAISER STEFAN J ET AL: "Natural isothiocyanates express antimicrobial activity against developing and mature biofilms of Pseudomonas aeruginosa", FITOTERAPIA, IDB HOLDING, MILAN, IT, vol. 119, 5 April 2017 (2017-04-05), pages 57 - 63, XP085058174, ISSN: 0367-326X, DOI: 10.1016/J.FITOTE.2017.04.006
- DATABASE WPI Week 201501, Derwent World Patents Index; AN 2014-V61328, XP002795530

## Beschreibung

Die Erfindung betrifft ein antimikrobielles Mittel auf Basis einer Wirkstoffkombination aus Isothiocyanaten.

Isothiocyanate kommen in einer Vielzahl von Pflanzen vor, vor allem solchen der Familie der Brassicaceae (Kreuzblütler) und der Kapuzinerkressen. Insbesondere sind zu nennen Kohlarten, wie Rosenkohl, Rotkohl, Brokkoli, Kohlrabi und dergleichen. Radieschen, Rettich, Senfsaat, Meerrettich und Kresse, sowie die Kapuzinerkresse. In diesen Pflanzen sind die Isothiocyanate Bestandteile des scharfen Prinzips. Sie werden häufig auch unter dem Begriff der Senföle zusammengefasst. Diese Inhaltsstoffe sind für ihre antibakterielle und fungizide Wirkung bekannt und werden seit langem in der Heilkunde genutzt, insbesondere in Form von Phytotherapeutika, die sie als Prodrugs zusammen mit Komponenten zu ihrer Aktivierung enthalten. Eine Wirksamkeit in der Krebstherapie wurde ebenfalls nachgewiesen.

Um ihre Wirkung zu entfalten, müssen diese Inhaltsstoffe in aktiver Form in den menschlichen Körper gelangen. Dem steht ihre außerordentliche Reaktivität und Empfindlichkeit entgegen. Isothiocyanate sind licht- und sauerstoffempfindlich und hydrolysieren in alkalischen wie sauren Medien. Die Verwendung in reiner Form ist deshalb problematisch.

Im Stand der Technik werden die Inhaltsstoffe in Form von Pflanzenpulvern eingesetzt, die die Inhaltsstoffe als Glucosinolate zusammen mit dem Enzym Myrosinase enthalten, wobei letzteres die Glucosinolate zu aktiven Isothiocyanaten hydrolysiert.

Die Glucosinolate als Vorläufer der Isothiocyanate sind in den einzelnen Pflanzen der Familie der Brassicaceae in unterschiedlichen Konzentrationen enthalten. Zudem enthalten die einzelnen Pflanzen verschiedene Glucosinolate. Die Glucosinolate werden bei der Zerkleinerung der Pflanzen freigesetzt und durch das ebenfalls freigesetzte Enzym Myrosinase in die freien Isothiocyanate umgewandelt. Beispiele für die Isothiocyanate sind Methylisothiocyanat, Phenylisothiocyanat, Benzylisothiocyanat, Allylisothiocyanat, Sulforaphan und dergleichen. Jede dieser Substanzen hat ihr eigenes Geschmacks- und Wirkungsspektrum.

Ein Problem der Verabreichung der Isothiocyanate in Form von Pflanzenprodukten ist die fehlende Standardisierung und Konfektionierung. Der Wirkstoffgehalt der verarbeiteten Pflanzen variiert und steht damit einer standardisierten Therapie entgegen. Zudem bedarf das Pflanzenprodukt der Aktivierung durch das Enzym, das den Wirkstoff erst freisetzt und damit aber auch der chemischen Zersetzung preisgibt. Der in einem Pflanzenprodukt enthaltene Anteil an Senfölen kommt deshalb in den Patienten nach der Einnahme nur zum Teil zugute.

Hinzu kommt, dass die einzelnen Isothiocyanate ein spezifisches Wirkspektrum haben und deshalb der Abstimmung auf den jeweils zu bekämpfenden Mikroorganismus bedürfen. Hilfreich wäre es, eine Wirkstoffkombination bereitzustellen, die gegen eine Vielzahl von Mikroorganismen wirksam ist.

Aus "Fachinformation Angocin Anti-Infekt N" vom 1. Juli 2015 ist ein antimikrobielles Mittel bekannt, das Allyl-, Benzyl- und Phenethylisothiocyanat in Form der Glucosinolate enthält. Die "OTC-Beratungskarte 76 Angocin Anti-Infekt N Filmtablette" vom 6. September 2017 offenbart ein antimikrobielles Mittel auf Basis von Pulver aus Kapuzinerkressenkraut und Meerrettichwurzel. In C. Andreas et al. "In-Vitro-Untersuchungen zur antibakteriellen Wirksamkeit einer Kombination aus Kapuzinerkressenkraut und Meerrettichwurzel", Arzneimittelforschung/Drug Research, 1. Januar 2006, S. 842 ff wird die antimikrobielle Aktivität dieses Mittels untersucht. S. J. Kaiser et al "Natural isothiocyanates express antimicrobial activity against developing and mature biofilms of Pseudomonas aeruginosa", Fitoterapia, Milan, IT, Bd. 119, 5:April 2017, S.57 ff offenbart, dass das phytomedizinische antimikrobielle Mittel Angocin seine Wirksamkeit aus den in Kapuzinerkressenkrautpulver und Meerrettichwurzelpulver vorhandenen Isothiocyanaten bezieht.

Aufgabe der Erfindung ist es, eine Wirkstoffkombination aus Isothiocyanaten bereitzustellen, die ein breites Wirkungsspektrum aufweist. Diese Wirkstoffkombination sollte zudem gegen chemische Veränderung/Zersetzung geschützt sein und erst am Wirkungsort freigesetzt werden.

Diese Aufgabe wird mit einer antimikrobiellen Zusammensetzung gelöst, die als Wirkstoffe eine Kombination aus 45 bis 55 Gew.-% Benzylisothiocyanat, 33 bis 43 Gew.-% Allylisothiocyanat und 7 bis 17 Gew.-% 2-Phenylethylisothiocyanat enthält, wobei sich die Gesamtmenge an Isothiocyanaten zu 100 Gew.-% ergänzt, an einen Trägerstoff gebunden ist und luftdicht in ein Verabreichungsmittel magensaftresistent eingeschlossen ist, wobei die an den inerten Trägerstoff gebundene Wirkstoffkombination pelletisiert und mit Chitosan mikroverkapselt ist.

Die erfindungsgemäß verwandten Wirkstoffe können ohne weiteres synthetisch erhalten werden. Eine Gewinnung aus den entsprechenden Pflanzen ist möglich, jedoch aufwendig.

Da jedes einzelne der Isothiocyanate pflanzlichen Ursprungs ein spezifisches Wirkungsspektrum hat, ist die Kombination mehrerer dieser Wirkstoffe sinnvoll, um ein möglichst breites Wirkungsspektrum zu erzielen. Die Kombination aus etwa 50 Gew.-% Benzylisothiocyanat, 38 Gew.-% Allylisothiocyanat und 12 Gew.-% 2-Phenylethylisothiocyanat hat sich als besonders wirksam gegen eine Vielzahl von Mikroorganismen erwiesen, darunter sowohl grampositive wie gramnegative Bakterien. Darüber hinaus sind Isothiocyanate auch gegen virale und Pilzinfektionen wirksam.

Die erfindungsgemäße Wirkstoffkombination ist an einen üblichen inerten Trägerstoff gebunden. Hier kommen insbesondere Gips, Calaincarbonat Kieselgur in feinteiliger Form und Kieselgel infrage, jedoch können auch beliebige andere Trägerstoffe, die sich in der Pharmazie bewährt haben, eingesetzt werden. Die Menge des Trägerstoffs bemisst sich nach dem Sorptionsvermögen für die flüssigen Wirkstoffe. Bevorzugt wird Kieselgur oder Kieselgel eingesetzt.

Die Verarbreichungsmittel sind in der Pharmazie übliche orale Mittel wie Tabletten, Dragees oder Kapseln, die den Anforderungen genügen.

Es ist wichtig, dass die Wirkstoffe luftdicht eingeschlossen sind. Isothiocyanate sind sehr reaktiv und insbesondere feuchtigkeits- und sauerstoffempfindlich. Der Luftabschluss erfolgt auf übliche Art und Weise durch Konfektionierung, etwa in einer Filmtablette oder einer luftdichten Kapsel. Grundsätzlich ist aber jede Verabreichungsform geeignet, die eine luftdichte Verpackung der erfindungsgemäßen Wirkstoffkombination gewährleistet.

Die erfindungsgemäße Form des Luftausschlusses ist die Mikroverkapselung der an den inerten Trägerstoff gebundenen Wirkstoffkombination. Diese wird pelletisiert und mit dem Verkapselungsmittel, Chitosan, überzogen. Die Pellets können dann in eine übliche Medikamentenkapsel gefüllt und damit verabreicht werden.

Eine solche Filmtablette oder Kapsel enthält zweckmäßigerweise 0,1 bis 100 mg der Wirkstoffkombination und insbesondere 0,5 bis 50 mg und besonders bevorzugt 1,0 bis 20 mg. Es hat sich gezeigt, dass eine Verabreichung dreimal täglich, vorzugsweise zu den Mahlzeiten, ausreichend ist. Die Verabreichung sollte über maximal eine Woche erfolgen.

Die Mengen an Trägerstoff betragen vorzugsweise 0,1 bis 1,0 g pro Filmtablette oder Kapsel, insbesondere 0,2 bis 0,5 g, abgestimmt auf die Menge der Wirkstoffe.

Wegen der Reaktionsfreudigkeit der Isothiocyanate ist die Wirkstoffkombination magensaftresistent tablettiert oder verkapselt. Dem Pharmazeuten sind hierfür geeignete Polymere, insbesondere Polymethacrylate (Eudragit), bekannt. Daraus gefertigte Filmtabletten oder Kapseln durchwandern den Magen unbeschädigt und lösen sich erst im Dünndarm unter Freisetzung der Wirkstoffkombination auf.

Zur Stabilisierung der Wirkstoffkombination kann das erfindungsgemäße Mittel zusätzlich Vitamin E enthalten. Vitamin E erhöht die Oxidationsbeständigkeit Chitosan dient als zusätzliche Barriere für Sauerstoff.

Die Menge an Vitamin E pro Filmtablette oder Kapsel beträgt zweckmäßigerweise 0,01 bis 0,5 g und insbesondere 0,01 bis 0,30 g. Chitosan ist in Mengen von 0,1 bis 0,5 g, vorzugsweise 0,1 bis 0,25 g in der Mikroverkapselung enthalten.

Zur Förderung der Resorption der Wirkstoffkombination im Darm eines Patienten kann das erfindungsgemäße Mittel mittelkettige Triglyceride enthalten. Bevorzugt ist Miglyol, dessen absorptionsfördernde Eigenschaft erprobt ist. Solche Triglyceride können in einer Menge von bis zu 1,0 ml in einer Kapsel zugegen sein.

Die Wirksamkeit einer erfindungsgemäßen Wirkstoffkombination mit 50 Gew.-% Benzylisothiocyanat, 37,9 Gew.-% Allylisothiocyanat und 12,1 Gew.-% 2-Phenyethylisothiocyanat wurde in vitro an Hand einer Reihe von pathogenen Keimen bestimmen. Die jeweils bestimmten minimalen Hemmkonzentrationen MHK 90 (in % v/v) und minimalen bakteriziden Konzentrationen MBK 90 (% v/v) sind in Tabelle 1 dargestellt.

| Strain /bacterium | MHK90 (% v/v) | MBK90 (% v/v) |
|---|---|---|
| Staphylococcus aureus | 0.004 | 0.5 |
| Streptococcus pneumoniae | 0.008 | 0.03 |
| S. pyogenes | 0.008 | 0.25 |
| Enterococcus faecalis | 0.015 | >1 |
| E. faecium | 0.015 | >1 |
| Pseudomonas aeruginosa | 0.03 | 0.06 |
| Escherichia coli | 0.015 | 0.25 |
| Klebsiella pneumoniae | 0.125 | 0.125 |
| Proteus vulgaris | 0.004 | 0.015 |
| Serratia marcescens | 0.004 | 0.004 |
| H. influenzae | ≤0,005 | ≤0.0005 |
| Candida spp. | 0.004 | 0.004 |

Isothiocyanate werden nach der Aufnahme vom Körper metabolisiert. Das Abbauprodukt N-Acetyl-5-(N-benzylthiocabamoyl)-L-cystein von Benzylisothiocyanat wird über die Niere ausgeschieden und kann im Urin quantitativ nachgewiesen werden. Bei freiwilligen Testpersonen ergab sich eine Ausscheidungsrate von 53 % (Mittelwert). Die Ausscheidung erfolgte schnell, mit einer maximalen Konzentration 2 bis 6 h nach der Verabreichung und war nach 10 bis 12 h abgeschlossen. Entsprechendes gilt für andere Isothiocyanate.

Die erfindungsgemäße Wirkstoffkombinationen wurden von den Testpersonen gut vertragen.

## Patentansprüche

1. Antimikrobielles Mittel, enthaltend als Wirkstoff eine Kombination aus 45 bis 55 Gew.-% Benzylisothiocyanat, 33 bis 43 Gew.-% Allylisothiocyanat und 7 bis 17 Gew.-% 2-Phenylethylisothiocyanat, wobei sich die Gesamtmenge der Wirkstoffe zu 100 Gew.-% ergänzt, **dadurch gekennzeichnet, dass** die Wirkstoffe an einen inerten Trägerstoff gebunden und luftdicht in ein Verabreichungsmittel magensaftresistent eingeschlossen sind, wobei die an dem inerten Trägerstoff gebundene Wirkstoffkombination pelletisiert und mit Chitosan mikroverkapselt ist.

2. Antimikrobielles Mittel nach Anspruch 1 in Form einer Filmtablette oder Kapsel.

3. Antimikrobielles Mittel nach einem der vorstehenden Ansprüche 1 oder 2, **dadurch gekennzeichnet, dass** es 0,1 bis 100 mg der Wirkstoffkombination enthält.

4. Antimikrobielles Mittel nach Anspruch 3, **dadurch gekennzeichnet, dass** es 0,5 bis 50 mg der Wirkstoffkombination enthält.

5. Antimikrobielles Mittel nach einem der vorstehenden Ansprüche, enthaltend etwa 50 Gew.-% Benzylisothiocyanat, 38 Gew.-% Allylisothiocyanat und 12 Gew.-% 2-Phenylethylisothiocyanat.

6. Antimikrobielles Mittel nach einem der vorstehenden Ansprüche, **dadurch gekennzeichnet, dass** der Trägerstoff Gips, Calciumcarbonat Kieselgur oder Kieselgel in feinteiliger Form ist.

7. Antimikrobielles Mittel nach einem der vorstehenden Ansprüche, **dadurch gekennzeichnet, dass** es Vitamin E enthält.

8. Antimikrobielles Mittel nach einem der vorstehenden Ansprüche, **dadurch gekennzeichnet, dass** es mittelkettige Triglyceride enthält, insbesondere ein Miglyol.

## Claims

1. Antimicrobial agent containing as active ingredient a combination of 45 to 55% by weight benzyl isothiocyanate, 33 to 43% by weight allyl isothiocyanate and 7 to 17% by weight 2-phenylethyl isothiocyanate, wherein the total amount of the active ingredients adds up to 100% by weight, **characterized in that** the active ingredients are bound to an inert carrier substance and are enclosed in an airtight manner in an administration agent resistant to gastric juice, wherein the active ingredient combination bound to the inert carrier substance is pelletized and microencapsulated with chitosan.

2. Antimicrobial agent according to claim 1 in the form of a film-coated tablet or capsule.

3. Antimicrobial agent according to any one of the preceding claims 1 or 2, **characterized in that** it contains 0.1 to 100 mg of the active ingredient combination.

4. Antimicrobial agent according to claim 3, **characterized in that** it contains 0.5 to 50 mg of the active ingredient combination.

5. Antimicrobial agent according to any one of the preceding claims, comprising about 50% by weight benzyl isothiocyanate, 38% by weight allyl isothiocyanate and 12% by weight 2-phenylethyl isothiocyanate.

6. Antimicrobial agent according to any one of the preceding claims, **characterized in that** the carrier material is gypsum, calcium carbonate diatomaceous earth or silica gel in finely divided form.

7. Antimicrobial agent according to any one of the preceding claims, **characterized in that** it contains vitamin E.

8. Antimicrobial agent according to any one of the preceding claims, **characterized in that** it contains medium-chain triglycerides, in particular a miglyol.

## Revendications

1. Agent antimicrobien contenant comme principe actif une combinaison de 45 à 55 % en poids d'isothiocyanate de benzyle, de 33 à 43 % en poids d'isothiocyanate d'allyle et de 7 à 17 % en poids d'isothiocyanate de 2-phényléthyle, dans lequel la quantité totale des principes actifs s'élevant à 100 % en poids %, **caractérisé en ce que** les principes actifs sont liés à un excipient inerte et enfermés hermétiquement dans un moyen d'administration résistant au suc gastrique, dans lequel la combinaison de principes actifs liée à l'excipient inerte étant granulée et microencapsulée avec du chitosane.

2. Agent antimicrobien selon la revendication 1 sous forme de comprimé pelliculé ou de capsule.

3. Agent antimicrobien selon l'une quelconque des revendications 1 ou 2 précedentes, **caractérisé en ce qu'**il contient 0,1 à 100 mg de la combinaison de principes actifs.

4. Agent antimicrobien selon la revendication 3, **caractérisé en ce qu'**il contient 0,5 à 50 mg de la combinaison de principes actifs.

5. Agent antimicrobien selon l'une des revendications précédentes, contenant environ 50 % en poids d'isothiocyanate de benzyle, 38 % en poids d'isothiocyanate d'allyle et 12 % en poids d'isothiocyanate de 2-phényléthyle.

6. Agent antimicrobien selon l'une des revendications précédentes, **caractérisé en ce que** le matériau support est du gypse, du carbonate de calcium de la terre de diatomées ou du gel de silice sous forme finement divisée.

7. Agent antimicrobien selon l'une des revendications précédentes, **caractérisé en ce qu'**il contient de la vitamine E.

8. Agent antimicrobien selon l'une des revendications précédentes, **caractérisé en ce qu'**il contient des triglycérides à chaîne moyenne, en particulier un miglyol.
